# EUROPÄISCHE PATENTSCHRIFT

(11) **EP 2 689 241 B1**
(45) Veröffentlichungstag und Bekanntmachung des Hinweises auf die Patenterteilung: **26.12.2018**
(21) Anmeldenummer: 12700812.6
(22) Anmeldetag: 17.01.2012
(51) Int. Cl.: G01N 25/56, G01K 13/00, G01N 7/14, A61B 5/00

(54) **VORRICHTUNG ZUR MESSUNG DER VON EINER HAUTOBERFLÄCHE DURCH DIFFUSION ABGEGEBENEN MENGE EINES STOFFES**
DEVICE FOR MEASURING THE AMOUNT OF A SUBSTANCE GIVEN OFF FROM A SKIN SURFACE BY DIFFUSION
ÉQUIPEMENT DE MESURE DE LA QUANTITÉ D'UNE SUBSTANCE LIBÉRÉE PAR DIFFUSION PAR UNE SURFACE DE LA PEAU

(30) Priorität: 25.03.2011 DE 202011004427 U
(43) Veröffentlichungstag der Anmeldung: 29.01.2014
(73) Patentinhaber: COURAGE + KHAZAKA ELECTRONIC GmbH, D-50829 Köln (DE)
(72) Erfinder: KHAZAKA, Gabriel, 50859 Köln (DE)
(74) Vertreter: dompatent von Kreisler Selting Werner - Partnerschaft von Patent- und Rechtsanwälten mbB
(86) Internationale Anmeldenummer: PCT/EP2012/050646
(87) Internationale Veröffentlichungsnummer: WO 2012/130488

(56) Entgegenhaltungen:
- DE-A1- 2 553 677
- DE-U1- 9 318 928
- US-A- 5 203 327
- US-A- 5 899 856
- US-A1- 2005 119 537
- SHAH J H ET AL: "Comparative evaporimetry in man", SKIN RESEARCH AND TECHNOLOGY, Bd. 11, Nr. 3, August 2005 (2005-08), Seiten 205-208, XP002671189, BLACKWELL PUBLISHING LTD GB DOI: 10.1111/J.1600-0846.2005.00099.X

## Beschreibung

Die Erfindung betrifft eine Vorrichtung zur Messung der von einer Hautoberfläche durch Diffusion abgegebenen Menge eines Stoffes.

Derartige Instrumente sind aus der DE 2553677 bekannt. Eine solche Vorrichtung zur Bestimmung der von einer Oberfläche durch Diffusion abgegebenen Menge eines Stoffes weist einen Messkopf auf. Dieser Messkopf ist eine offene Messkapsel, in der mindestens zwei Sensoren angeordnet sind, wobei die Sensoren in unterschiedlichen Abständen zu der zu untersuchenden Hautfläche angeordnet sind. Mit diesen Instrumenten lässt sich vorzugsweise der transepidermale Wasserverlust messen, also die Feuchtigkeit, die von der Haut abgesondert wird.

Die Messung mit den oben genannten Geräten hat den Nachteil, dass die Menge der abgegebenen Feuchtigkeit sehr gering ist. Daher sind die Geräte ungenau. Der Erfindung liegt daher die Aufgabe zugrunde, eine Vorrichtung zur Messung des von einer Hautoberfläche durch Diffusion abgegebenen Menge eines Stoffes zu schaffen, bei der die Genauigkeit der Messung erhöht wird.

Zur Lösung dieser Aufgabe dienen die Merkmale des Anspruchs 1. Die Erfindung sieht in vorteilhafter Weise vor, dass der Messkopf mindestens zwei Messeinrichtungen aufweist, die voneinander beabstandet angeordnet sind, wobei die insgesamt zwei Messeinrichtungen unabhängig voneinander beweglich sind, so dass sie sich beim Aufsetzen auf die Hautoberfläche an die Krümmung der Hautoberfläche anpassen können, wobei die jeweiligen Messeinrichtungen jeweils eine offene Messkapsel aufweisen, wobei die Messkapsel mindestens zwei Öffnungen aufweist, wobei eine der mindestens zwei Öffnungen auf der zu untersuchenden Hautoberfläche plazierbar ist, wobei an einem Griff mindestens eine Kopplungseinrichtung vorgesehen ist, und wobei an dieser Kopplungseinrichtung die Messeinrichtungen angeordnet sind. Dies hat den Vorteil, dass die Messeinrichtungen zum Messen gleichzeitig auf der zu untersuchenden Hautoberfläche platzierbar sind, selbst wenn die Hautoberfläche eine Krümmung aufweist, d.h. dass die Hautoberfläche uneben ist.

Dies hat ferner den Vorteil, dass die Messung wesentlich genauer ist, da an mehreren vergleichbaren Stellen der Haut gleichzeitig gemessen werden kann und die Messwerte gemittelt werden können. Ferner hat die vorliegende Erfindung den Vorteil, dass unterschiedliche benachbarte Bereiche des Hautabschnitts untersucht werden können, wobei die unterschiedlichen Stellen vorher beispielsweise mit unterschiedlichen Hautcremes behandelt werden können, wobei bei dieser Messung die Messwerte der jeweiligen Messeinrichtungen einzeln ausgegeben werden. Auf diese Weise kann eine Messung gleichzeitig unter unterschiedlichen Bedingungen an vergleichbaren Hautstellen durchgeführt werden.

Vorzugsweise weist die Vorrichtung mindestens drei Messeinrichtungen auf. Es können auch mindestens vier oder mindestens fünf Messeinrichtungen vorgesehen sein.

Die jeweils mindestens zwei Messeinrichtungen können jeweils mindestens einen Rotationsfreiheitsgrad aufweisen, so dass sie unabhängig voneinander beweglich sind, wobei beim Messen alle Messeinrichtungen gleichzeitig auf einer eine Krümmung aufweisende Hautoberfläche plazierbar sind. Auch können die jeweils mindestens zwei Messeinrichtungen jeweils mindestens zwei Rotationsfreiheitsgrade aufweise, so dass sie unabhängig voneinander beweglich sind. Auch können die mindestens zwei Messeinrichtungen drei Rotationsfreiheitsgrade aufweisen, so dass sie unabhängig voneinander beweglich sind.

Es kann mindestens ein Rückstellelement vorgesehen sein, dass die Messeinrichtungen nach dem Messen, wenn sie nicht im Kontakt mit der zu untersuchenden Hautoberfläche sind, wieder in die Ausgangslage zurückstellen. Dies hat den Vorteil, dass keine zusätzliche Hand benötigt wird, um den Messkopf mit den mindestens zwei Messeinrichtungen auf der Haut zu platzieren.

Die mindestens zwei Messeinrichtungen können mit mindestens einer Kopplungseinrichtung verbunden sein. Die jeweiligen Messeinrichtungen können jeweils über eine Kopplungseinrichtung mit der Vorrichtung verbunden sein. Auch können die mindestens zwei Messeinrichtungen über die mindestens eine Kopplungseinrichtung miteinander verbunden sein.

Die mindestens eine Kopplungseinrichtung kann zumindest teilweise aus elastischem Kunststoff bestehen. Dieser elastische Kunststoff hat den Vorteil, dass die Messeinrichtungen nach dem Messen, wenn sie nicht mehr in Kontakt mit der zu untersuchenden Hautoberfläche sind, sich wieder in die Ausgangslage zurückstellen.

Die mindestens eine Kopplungseinrichtung kann ein Grundelement aufweisen, wobei die mindestens zwei Messeinrichtungen über jeweils mindestens eine Gelenkverbindung mit mindestens einem Rotationsfreiheitsgrad mit dem Grundelement gelenkig verbunden sind. Alternativ können die mindestens zwei Messeinrichtungen mindestens eine Gelenkverbindung mit mindestens zwei Rotationsfreiheitsgraden mit dem Grundelement gelenkig verbunden sein. Auch können die mindestens zwei Messeinrichtungen über mindestens eine Gelenkverbindung mit drei Rotationsfreiheitsgraden mit dem Grundelement gelenkig verbunden sein.

Die jeweilige Gelenkverbindung kann pro Rotationsfreiheitsgrad ein separates Gelenk aufweisen, wobei die Gelenke einer Gelenkverbindung miteinander gekoppelt sind.

Alternativ kann die jeweilige Gelenkverbindung mit einem bestimmten Rotationsfreiheitsgrad ein einzelnes Gelenk mit dem bestimmten Rotationsfreiheitsgrad aufweisen. Ein Gelenk mit zwei Rotationsfreiheitsgraden ist z.B. ein Kardangelenk und ein Gelenk mit drei Rotationsfreiheitsgraden ist z.B. ein Kugelgelenk.

Es kann in der jeweiligen Gelenkverbindung pro Freiheitsgrad eine Rückstellfeder vorgesehen sein, die die jeweilige Messeinrichtung wieder in die eine Ausgangslage bezogen auf das Grundelement zurückstellen.

Der Abstand zwischen den Messeinrichtungen kann einstellbar sein. Dies hat den Vorteil, dass unterschiedliche Hautstellen, die einen unterschiedlichen Abstand voneinander aufweisen, untersucht werden können.

Zwischen den jeweiligen Messeinrichtungen und dem Grundelement kann ein Verstellarm angeordnet sein, so dass der Abstand zwischen den Messeinrichtungen verstellbar ist.

Dieser Verstellarm kann zwischen der Gelenkverbindung und der jeweiligen Messeinrichtung angeordnet sein. Alternativ kann der Verstellarm zwischen dem Grundelement und der Gelenkverbindung angeordnet sein. Als weitere Alternative kann der Verstellarm ein Teil der Gelenkverbindung sein.

Die jeweiligen Messeinrichtungen können jeweils eine offene Messkapsel aufweisen, wobei die Messkapsel mindestens zwei Öffnungen aufweist, wobei eine der mindestens zwei Öffnungen auf der zu untersuchenden Hautoberfläche platzierbar ist.

In der Messkapsel können mindestens zwei Sensoren angeordnet sein, wobei die Sensoren beim Messen in unterschiedlichen Abständen von der zu untersuchenden Hautoberfläche angeordnet sind.

Weiter kann ein Gerät mit einer Vorrichtung zur Messung der von der Hautoberfläche durch Diffusion abgebende Menge eines Stoffs vorgesehen sein, wobei das Gerät einer Auswerteeinheit aufweist, die mit der Vorrichtung verbunden ist und die Messergebnisse der Messeinrichtung erhält, wobei die Auswerteeinheit einen Durchschnittsmesswert der einzelnen Messeinrichtung berechnet und ausgibt.

Alternativ kann die Auswerteeinheit die Messwerte der einzelnen Messeinrichtungen einzeln ausgeben und anzeigen, zu welcher Messeinrichtung das jeweilige Messergebnis gehört.

Im Folgenden wird unter Bezugnahme auf die Zeichnungen Ausführungsbeispiele der Erfindung näher erläutert.

Es zeigen schematisch
- Fig. 1: ein erstes Ausführungsbeispiel in der Draufsicht,
- Fig. 2: das Ausführungsbeispiel aus Figur 1 in der Seitenansicht,
- Fig. 3a: ein weiteres Ausführungsbeispiel mit einem Kardangelenk,
- Fig. 3b: ein weiteres Ausführungsbeispiel mit einem Kugelgelenk,
- Fig. 3c: ein weiteres Ausführungsbeispiel einer Gelenkverbindung,
- Fig. 3d: ein weiteres Ausführungsbeispiel mit einem elastischen Element,
- Fig. 4a: ein weiteres Ausführungsbeispiel mit Verstellarmen,
- Fig. 4b: das Ausführungsbeispiel aus Fig. 4a in einer anderen Stellung,
- Fig. 5: ein weiteres Ausführungsbeispiel,
- Fig. 6: das Ausführungsbeispiel aus Figur 5 in der Seitenansicht,
- Fig. 7: ein weiteres Ausführungsbeispiel mit hintereinander angeordneten Messeinrichtungen,
- Fig. 8: die Messeinrichtungen auf einer Hautoberfläche,
- Fig. 9: die Messeinrichtungen auf einer anderen Hautoberfläche.

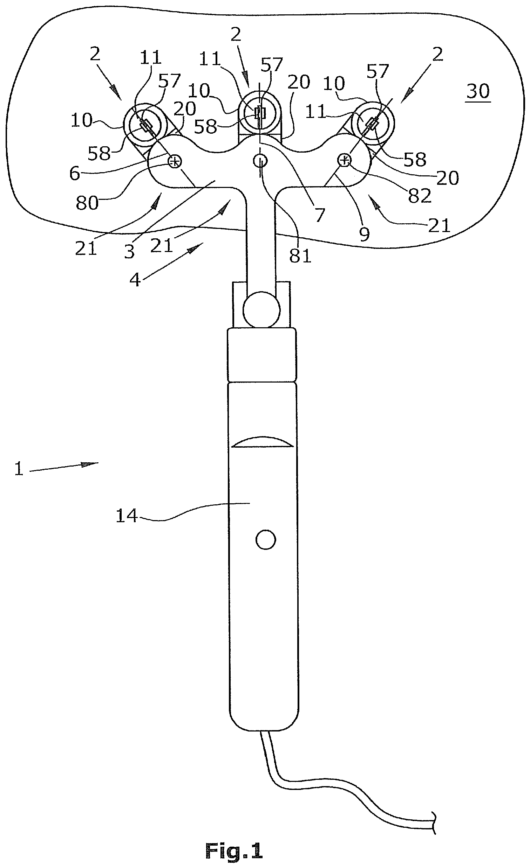

Fig. 1 zeigt ein erstes Ausführungsbeispiel der erfindungsgemäßen Vorrichtung 1 zur Messung der von einer Hautoberfläche durch Diffusion abgebenden Menge eines Stoffes.

Die Vorrichtung 1 weist einen Griff 14 auf. An dem Griff 14 ist eine Kopplungseinrichtung 4 befestigt. An dieser Kopplungseinrichtung 4 sind Messeinrichtungen 2 angeordnet.

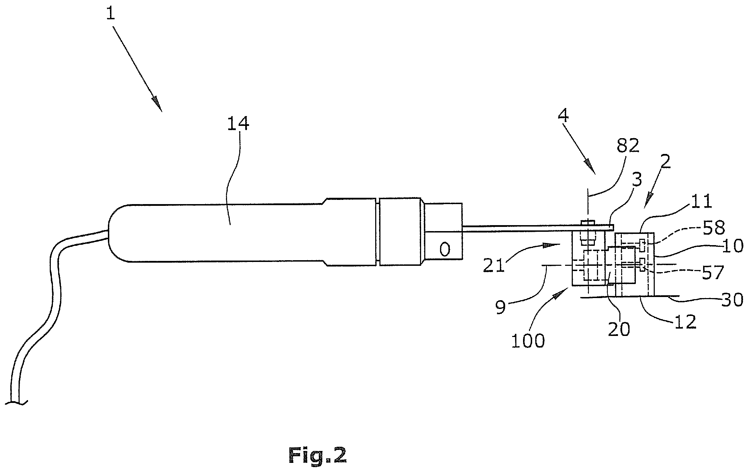

Die Messeinrichtungen 2 weisen eine offene Messkapsel 10 auf, wobei die Messkapsel 10 mindestens zwei Öffnungen 11, 12 aufweist, wobei eine der mindestens zwei Öffnungen 12 auf der zu untersuchenden Hautoberfläche 30 platzierbar ist. Die offene Messkapsel 10 gemäß dem Ausführungsbeispiel aus Fig. 1 und 2 weist mindestens zwei Sensoren 57, 58 auf, wobei die Sensoren 57, 58 beim Messen, d. h. wenn die Öffnungen 12 auf einer zu untersuchenden Hautoberfläche 30 aufliegen, in unterschiedlichen Abständen von der zu untersuchenden Hautoberfläche 30 angeordnet sind. Die Sensoren können die Temperatur und die relative Feuchtigkeit der Luft messen. Aus diesen Werten kann der transepidermale Wasserverlust der Haut bestimmt werden. Die Messergebnisse der Sensoren können über elektrische Leitungen, die im Inneren der Vorrichtung verlaufen und in den Figuren nicht dargestellt sind, an eine Auswerteeinheit gesendet werden. Alternativ können jedoch auch andere auf der Hautoberfläche plazierbare Messeinrichtungen verwendet werden. Diese Messeinrichtungen müssen lediglich die von einer Hautoberfläche durch Diffusion abgebende Menge eines Stoffes messen können.

Die Auswerteeinheit kann einen Durchschnittsmesswert aus den Messwerten der einzelnen Messeinrichtung 2 berechnen und ausgeben. Alternativ kann die Auswertereinheit die Messwerte der einzelnen Messeinrichtung 2 einzeln ausgeben und anzeigen, zu welcher Messeinrichtung 2 das jeweilige Messergebnis gehört.

In Fig. 2 ist die Vorrichtung aus Fig. 1 in der Seitenansicht dargestellt. Allerdings ist der Übersichtlichkeit halber in Fig. 2 nur eine Messeinrichtung 2 dargestellt. Die in den Fign. 1 und 2 dargestellte Kopplungseinrichtung 4 weist ein Grundelement 3 und Gelenkverbindungen 21 auf, wobei die Messeinrichtungen 2 jeweils über eine Gelenkverbindung 21 mit dem Grundelement 3 verbunden sind.

Die Messeinrichtungen 2 können aufgrund der Gelenkverbindungen 21 im Bezug zu dem Grundelement 3 jeweils um eine erste Ache 6, 7 bzw. 9 und jeweils um eine orthogonal zu der ersten Achse verlaufenden zweiten Achse 80, 81 und 82 verschwenkt werden. Die Messeinrichtungen 2 können sich, wenn sie jeweils um die Achse 6, 7 und 9 und/oder die Achse 80, 81, 82 verschwenkt werden, unabhängig voneinander bewegen, so dass sie zum Messen gleichzeitig auf einer makroskopisch unebenen Hautoberfläche plazierbar sind. Die Messeinrichtungen 2 sind unabhängig voneinander beweglich, so dass sie sich beim Aufsetzen auf die zu untersuchende Hautoberfläche an die Krümmung der Hautoberfläche anpassen können

Die jeweils ersten Achsen 6, 7 und 9 verlaufen in dem dargestellten Ausführungsbeispiel alle in einer Ebene. Die ersten Achsen 6, 7 und 9, um die die jeweiligen Messeinrichtungen 2 schwenkbar sind, verlaufen parallel zu der Ebene, in der die jeweilige auf der Hautoberfläche plazierbare Öffnung 12 der Messeinrichtungen 2 verläuft.

Die jeweiligen Achsen 80, 81 und 82 verlaufen orthogonal zu den Achsen 6, 7 und 9.

In dem Fall, in dem die Messeinrichtungen 2 auf einer ebenen Oberfläche plaziert sind, verlaufen die Achsen 6, 7 und 9 in der Horizontalen und die Achsen 80, 81 und 82 in der Vertikalen.

Im Folgenden wird bezogen auf Fig. 2 eine Gelenkverbindung 21 näher erläutert. Die Gelenkverbindung 21 weist ein Zwischenelement 100 auf. Das Grundelement 3 ist über das Zwischenelement 100 mit der entsprechenden Messeinrichtung 2 verbunden. Das Grundelement 3 kann um die Achse 82 bezogen auf das Zwischenelement 100 verschwenkt werden. Die Messeinrichtung 2 kann um die Achse 9 bezogen auf das Zwischenelement 100 verschwenkt werden. Somit kann die Messeinrichtung 2 bezogen auf das Grundelement 3 um die Achse 9 und um die Achse 82 verschwenkt werden. Diese beiden Schwenkbewegungen können sich überlagern. Die Messeinrichtung 2 kann somit in Bezug auf zwei Rotationsfreiheitsgrade verschwenkt bzw. bewegt werden. Die Messeinrichtung 2 kann sich somit an eine makroskopisch unebene Oberfläche bzw. an eine Krümmung einer Oberfläche anpassen. Die anderen beiden Messeinrichtungen 2 können auf die gleiche Art und Weise um die Achsen 7 und 81 bzw. 6 und 80 verschwenkt werden.

Die Gelenkverbindungen 21 weisen somit zwei Rotationsfreiheitsgrade auf, so dass die Messeinrichtungen 2 im Bezug zu dem Grundelement 3 bezogen auf zwei Rotationsfreiheitsgrad unabhängig voneinander bewegbar sind.

Ein Objekt kann in einem Raum bezüglich drei Rotationsfreiheitgraden und drei Translationsfreiheitsgraden bewegbar sein. D.h. das Objekt weist drei Rotationsfreiheitsgerade und drei Translationsfreiheitsgerade auf. Wenn ein Objekt bezüglich drei Rotationsfreiheitsgraden bewegbar ist bzw. drei Rotationsfreiheitsgerade aufweist, so kann diese Objekt um drei orthogonal zueinander verlaufende Achse verschwenkt werden, wobei jeweils zwei der drei Achsen in einer Ebene verlaufen und die dritte Achse orthogonal zu dieser Ebene verläuft. Wenn dieses Objekt zusätzlich in Bezug zu den drei Translationsfreiheitsgraden bewegbar ist bzw. drei Translationsfreiheitsgerade aufweist, so kann das Objekt entlang dieser drei Achsen bewegbar sein. Wenn ein Objekt bezüglich zweier Rotationsfreiheitsgrade bewegbar ist, so kann das Objekt um zwei orthogonal zueinander verlaufenden Achsen bewegbar sein.

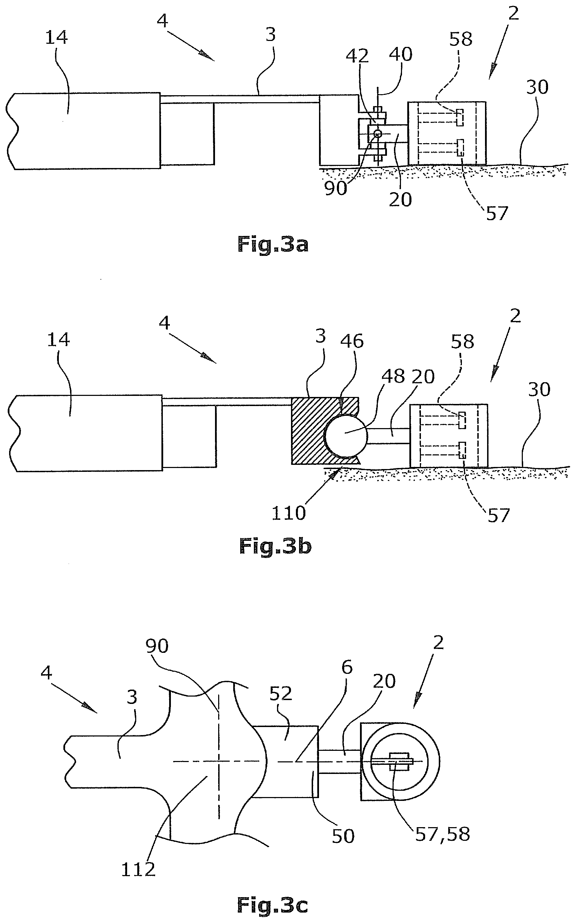

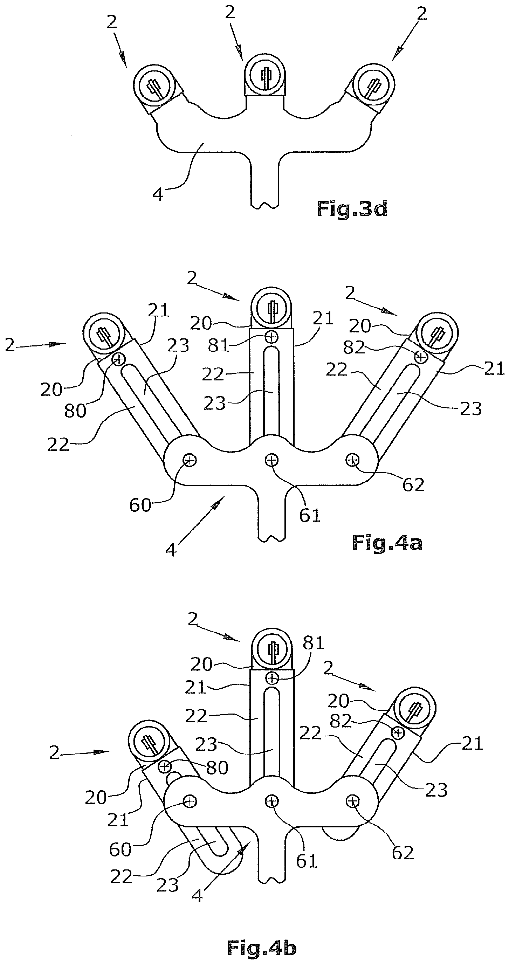

Alternativ kann die Gelenkverbindung 21 des ersten Ausführungsbeispiels einen Rotationsfreiheitsgrad bzw. drei Rotationsfreiheitsgrade aufweisen. Wenn die Gelenkverbindung 21 zwei Rotationsfreiheitsgrade aufweist, kann die Gelenkverbindung 21 auch ein Kardangelenk sein. Ein Kardangelenk ist in Fig. 3 dargestellt.

Bei dem Kardangelenk gemäß Fig. 3a ist das Grundelement über ein Zwischenelement 42 mit der jeweiligen Messeinrichtung 2 verbunden. Das Zwischenelement 42 weist zwei orthogonal zueinander verlaufende Wellen auf, wobei die Achsen der Wellen die Achsen 90 und 40 sind. Das Grundelement 3 kann um die Achse 40 bezogen auf das Zwischenelement 42 verschwenkt werden. Die Messeinrichtung 2 kann um die Achse 90 bezogen auf das Zwischenelement 42 verschwenkt werden. Somit kann die Messeinrichtung 2 bezogen auf das Grundelement um die Achse 90 und um die Achse 40 verschwenkt werden. Diese beiden Schwenkbewegungen können sich überlagern. Die Messeinrichtung 2 kann somit in Bezug auf zwei Rotationsfreiheitsgrade verschwenkt bzw. bewegt werden. Die Messeinrichtung 2 kann sich somit an die Krümmung einer Oberfläche anpassen. Dadurch, dass jede der Messeinrichtung 2 ein solches Kardangelenk aufweist, können die Messeinrichtungen 2 unabhängig voneinander bewegt werden, so dass sich jede der Messeinrichtungen 2 beim Aufsetzen auf die Hautoberfläche an die Krümmung der Hautoberfläche anpassen kann. Die Achse 90 in dem Ausführungsbeispiel gemäß Fig. 3a verläuft ebenfalls, so wie die Achsen 6, 7 und 9 des Ausführungsbeispiels auf Fign. 1 und 2, in der Ebene der Öffnung 12. Allerdings verliefe die Achse 90, wenn sie in einer Messeinrichtung 2 mit einer der Achsen 6, 7 oder 9 angeordnet wäre, orthogonal zu dieser Achse 6, 7 oder 9.

Die Achse 40 verläuft orthogonal zu der Achse 90 und im Gegensatz zu den Achsen 80, 81 und 82 des Ausführungsbeispiels auf Fign. 1 und 2 verläuft die Achse 90 in jeder Stellung der Messeinrichtung 2 jeweils orthogonal zu der Ebene, in der die Öffnung 12 angeordnet ist.

Alternativ können die Gelenkverbindungen 21 auch ein Kugelgelenk 110 aufweisen. Ein solches ist in Fig. 3b dargestellt. Ein Kugelgelenk weist drei Rotationsfreiheisgrade auf. Das Grundelement 3 weist eine kugelförmige Aufnahmeeinrichtung 46 für das mit der jeweiligen Messeinrichtung 2 verbundene Kugelelement 48 auf. Die Messeinrichtung 2 kann bezogen auf das Grundelement 3 um drei orthogonal zueinander verlaufenden Rotationsachsen rotieren, wobei sich die Rotationsbewegungen einander überlagern können. Die Messeinrchtung 2 kann sich somit bezogen auf das Grundelement 3 bezüglich dreier Rotationsfreiheitsgrade bewegen. Dadurch, dass jede der Messeinrichtung 2 ein solches Kugelgelenk aufweist, können die Messeinrichtungen 2 unabhängig voneinander bewegt werden, so dass sich jede der Messeinrichtungen 2 beim Aufsetzen auf die Hautoberfläche an die Krümmung der Hautoberfläche anpassen kann, so dass die jeweilige Öffnung 12 auf der Hautoberfläche plazierbar ist.

In Fig. 3c ist eine weitere Alternative einer Gelenkverbindung 21 dargestellt. Die Gelenkverbindung 21 gemäß Fig. 3c weist pro Rotationsfreiheitsgrad ein Gelenk auf, wobei diese Gelenke miteinander gekoppelt. Die Gelenke sind über das Element 52 miteinander gekoppelt sind. Das Grundelement 3 kann um die Achse 90 des Gelenks 112 bezogen auf das Element 52 verschwenkt werden. Die Messeinrichtung 2 kann bezogen auf das Element 52 um die Achse 6 des Gelenks 50 verschwenkt werden. Es sind lediglich die Achsen 6, 90 der Gelenke 50, 112 dargestellt. Die Achsen 6 und 90 verlaufen orthogonal zueinander, wobei beiden Achsen 6, 90 parallel zu der Ebene verlaufen, in der die Öffnung 12 verläuft. Die in Fig. 3c dargestellte Gelenkverbindung weist zwei Rotationsfreiheitsgrade auf.

Bei den in den Fign. 1-3c dargestellten Gelenkverbindungen können auch Rückstellelemente vorgesehen sein, die die Messeinrichtungen 2 nach dem Messen, wenn die Messeinrichtungen nicht mehr auf der Hautoberfläche plaziert sind, wieder in eine Ausgangslage zurückschwenken. Als Rückstellelemente können beispielsweise Rückstellfedern verwendet werden. Diese Rückstellfedern können beispielsweise Torsionsfedern sein, die konzentrisch zu der jeweiligen Achse angeordnet ist.

Als Alternative zu Gelenkverbindungen kann die Kopplungseinrichtung 4 aus einem elastischen Material bestehen. Ein solches Ausführungsbeispiel ist in Fig. 3d dargestellt. Das elastische Material kann beispielsweise Gummi sein. Die Messeinrichtungen 2 sind an der elastischen Kopplungseinrichtung 4 befestigt. Die Messeinrichtungen 2 können beispielsweise mittels einer Klebeverbindung an der elastischen Kopplungseinrichtung 4 befestigt sein. Dadurch, dass die Kopplungseinrichtung 4 elastisch ausgeführt ist, können sich die Messeinrichtungen 2 unabhängig voneinander bewegen und beim Aufsetzen auf eine Hautoberfläche an die Krümmung der Hautoberfläche angepasst werden.

Als weitere Alternative kann die mindestens eine Kopplungseinrichtung 4, damit die Messeinrichtungen 2 an eine Krümmung der Hautoberfläche anpassbar sind, Gelenkverbindungen und zusätzlich elastische Elemente aufweisen. So kann beispielsweise bei den in Fig. 1 bis 3c dargestellten Ausführungsbeispielen jeweils die Elemente 20 der Messeinrichtungen 2 elastisch aufgeführt sein. Die Elemente 20 können aus einem elastischen Material, wie z.B. Gummi, bestehen. Die Messeinrichtung 2 können somit um die bereits beschriebenen Achsen rotiert werden und zusätzlich können sie aufgrund des elastischen Elementes 20 bewegbar sein, so dass sie beim Aufsetzen auf die Hautoberfläche optimal an die Krümmung der Hautoberfläche anpassbar sind. Das Material der elastischen Elemente 20 kann derart gewählt sein, dass die Messeinrichtungen aufgrund des elastischen Elements 20 an kleine Krümmungen der Hautoberfläche anpassbar sind und aufgrund der Gelenkverbindung 21 an größere Krümmungen der Hautoberfläche anpassbar sind.

In Fign. 4a und 4b ist ein weiteres Ausführungsbeispiel dargestellt. Es unterscheidet sich von dem Ausführungsbeispiel aus den Fign. 1 und 2 dadurch, dass Verstellarme 22 vorgesehen sind. Die Verstellarme 22 sind Teil der Kopplungseinrichtung 4. Die Verstellarme 22 können bezüglich des Grundelementes 3 verstellt werden. So können die Verstellarme 22 z.B. um jeweils eine Achse 60, 61, 62 verschwenkt werden. Zudem können die Verstellarme translatorisch in Bezug zu dem Grundelement 3 entlang der Langlöcher 23 verstellt werden, so dass der Abstand der Messeinrichtungen 2 zu dem Grundelement 3 verstellbar bzw. einstellbar ist. Auf diese Weise kann auch der Abstand zwischen den Messeinrichtungen 2 verändert werden. Der Abstand zwischen den Messeinrichtungen 2 ist somit einstellbar bzw. verstellbar. In Fig. 4b sind die Verstellarme 22 in einer anderen Stellung dargestellt als in Fig 4a.

Dadurch, dass der Abstand zwischen dem Messeinrichtungen 2 veränderbar ist, können unterschiedliche Hautabschnitte bei einer Messung untersucht werden. Diese unterschiedlichen Hautabschnitte können vor der Messung z.B. mit unterschiedlichen Hautcremes behandelt werden.

Die in den Fign. 4a und 4b dargestellten Messeinrichtungen 2 lassen sich, genauso wie in den Fign. 1 und 2 beschrieben, aufgrund der Gelenkverbindung 21 in Bezug zu dem jeweiligen Verstellarm 22 verschwenken. Auf diese Weise lassen sich die Messeinrichtungen 2 bezogen auf das Grundelement 3 und bezogen auf den jeweiligen Verstellarm 22 um zwei Rotationsfreiheitsgrade bewegen. Auf diese Weise können sich die Messeinrichtungen 2 unabhängig voneinander bewegen und sich flexibel an die Krümmung einer Hautoberfläche anpassen.

Es versteht sich, dass unterschiedlichen Gelenkverbindungen, z.B. solche wie in den Fign. 3a-3c dargestellt, in dem Ausführungsbeispiel gemäß Fig. 4a und 4b verwendet werden können. Auch kann eine Kombination aus Gelenkverbindungen und elastischen Elementen verwendet werden. So kann z.B. eine Gelenkverbindung zwischen Verstellarm 22 und Grundelement 3 vorgesehen sein und das Element 20 kann elastisch ausgeführt sein. Als weitere Alternative können auch die Verstellarme 22 elastisch ausgeführt sein.

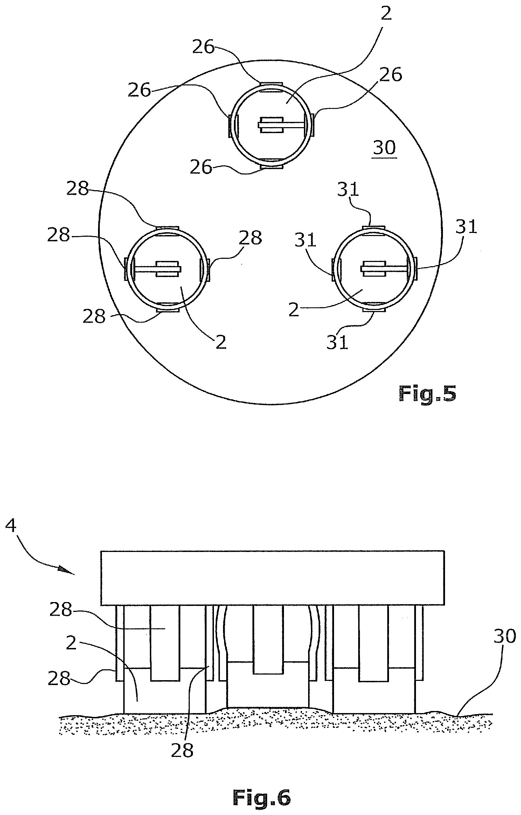

In Figur 5 und 6 ist ein weiteres Ausführungsbeispiel dargestellt. Die Messeinrichtungen 2 sind beabstandet voneinander angeordnet. Die Messeinrichtungen 2 sind über Kopplungseinrichtungen 26, 28, 31 mit der Vorrichtung verbunden. Die Kopplungseinrichtungen 26, 28, 31 sind elastische Elemente, z.B. elastische Kunststoffelemente, wie z.B. Gummielemente.

Wie in Figur 6 dargestellt können die Messeinrichtungen 2 beim Aufsetzen auf die Hautoberfläche an die Krümmung der Hautoberfläche bzw. an die unebene Hautoberfläche anpassen, da die mindestens zwei Messeinrichtungen 2 aufgrund der elastischen Kopplungseinrichtungen 26, 28, 31 unabhängig voneinander beweglich sind. Wenn die Messeinrichtungen 2 nicht auf einer Hautoberfläche plaziert sind, werden die Messeinrichtungen 2 wieder in eine Ausgangsposition zurückgestellt, da die Kopplungseinrichtungen 26, 28, 31 elastisch sind. Die Kopplungseinrichtungen 26, 28, 31 sind somit auch Rückstellelemente.

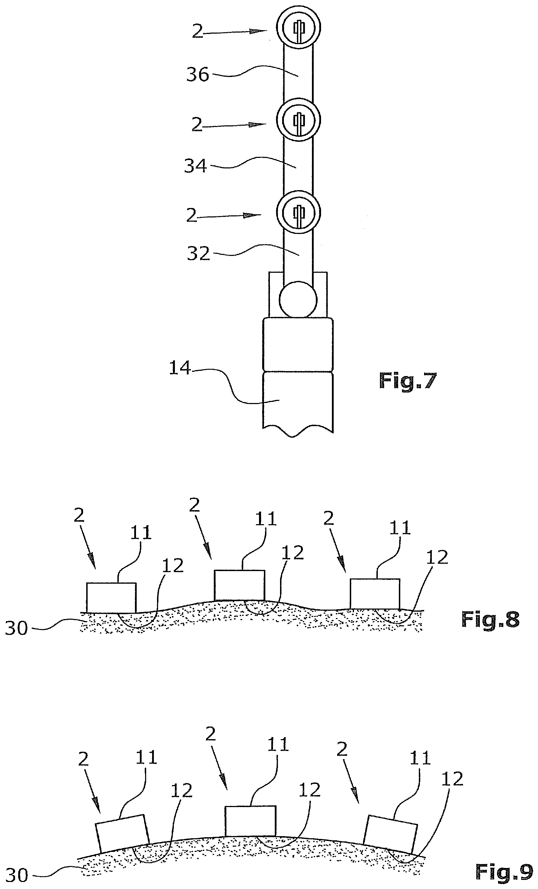

In Figur 7 ist ein weiteres Ausführungsbeispiel dargestellt, bei dem die Messeinrichtungen 2 hintereinander angeordnet sind. Es ist jeweils eine Messeinrichtung 2 mit einer anderen Messeinrichtung 2 verbunden, wobei nur die erste Messeinrichtung 2 über die Kopplungseinrichtung 32 mit dem Grundelement 3 verbunden ist. Die zweite Messeinrichtung 2 ist über die Kopplungseinrichtung 34 mit der ersten Messeinrichtung 2 verbunden und die dritte Messeinrichtung 2 ist über die Kopplungseinrichtung 36 mit der zweiten Messeinrichtung 2 verbunden. Die in Fig. 7 dargestellten Kopplungseinrichtungen 32,34, 36 sind elastisch ausgeführt. Die Kopplungseinrichtungen 32, 34, 36 können jedoch auch Gelenkverbindungen sein. Diese Gelenkverbindungen können z.B. Kardangelenke aufweisen.

In Figuren 8 und 9 sind jeweils die auf einer Hautoberfläche plazierten Messeinrichtungen 2 einer erfindungsgemäßen Vorrichtung dargestellt. Die Hautoberfläche kann beispielsweise die Hautoberfläche der Haut, des Arms oder des Gesichts sein. Die Messeinrichtungen 2 sind an die Krümmung der Hautoberfläche angepasst. In den Figuren 8 und 9 sind jedoch von der Vorrichtung nur die Messeinrichtungen 2 dargestellt. Es ist in den Figuren 8 und 9 dargestellt, wie die Messeinrichtungen 2 sich an die unebene Hautoberfläche anpassen können. Die untere Öffnung 12 liegt dabei plan auf dem entsprechenden zu untersuchenden Hautabschnitt auf.

## Patentansprüche

1. Vorrichtung (1) zur Messung der von einer Hautoberfläche (30) durch Diffusion abgegebenen Menge eines Stoffes, mit
einem auf der Hautoberfläche (30) plazierbaren Messkopf,
wobei der Messkopf mindestens zwei Messeinrichtungen (2) aufweist, die voneinander beabstandet angeordnet sind, wobei die insgesamt mindestens zwei Messeinrichtungen (2) unabhängig voneinander beweglich sind, so dass sie beim Aufsetzen auf die Hautoberfläche an die Krümmung der Hautoberfläche (30) anpassbar sind, wobei die jeweiligen Messeinrichtungen (2) jeweils eine offene Messkapsel (10) aufweisen, wobei die Messkapsel (10) mindestens zwei Öffnungen (11,12) aufweist, wobei eine der mindestens zwei Öffnungen (12) auf der zu untersuchenden Hautoberfläche (30) plazierbar ist, wobei an einem Griff (14) mindestens eine Kopplungseinrichtung (4) vorgesehen ist, und wobei an dieser Kopplungseinrichtung (4) die Messeinrichtungen (2) angeordnet sind.

2. Vorrichtung nach Anspruch 1, **dadurch gekennzeichnet, dass** die jeweils mindestens zwei Messeinrichtungen (2) bezogen auf mindestens einen Rotationsfreiheitsgrad unabhängig voneinander beweglich sind, so dass beim Messen alle Messeinrichtungen (2) gleichzeitig auf einer unebenen Hautoberfläche (30) plazierbar sind.

3. Vorrichtung nach Anspruch 1 oder 2, **dadurch gekennzeichnet, dass** die jeweils mindestens zwei Messeinrichtungen (2) bezogen auf mindestens zwei Rotationsfreiheitsgrade unabhängig voneinander beweglich sind.

4. Vorrichtung nach einem der Ansprüche 1 bis 3, **dadurch gekennzeichnet, dass** die jeweils mindestens zwei Messeinrichtungen (2) bezogen auf drei Rotationsfreiheitsgrade unabhängig voneinander beweglich sind.

5. Vorrichtung nach einem der Ansprüche 1 bis 4, **dadurch gekennzeichnet, dass** mindestens ein Rückstellelement vorgesehen ist, dass die Messeinrichtungen (2) nach dem Messen, wenn sie nicht in Kontakt mit der zu untersuchenden Hautoberfläche (30) sind, wieder in die Ausgangslage zurückstellt.

6. Vorrichtung nach Anspruch 1, **dadurch gekennzeichnet, dass** die Kopplungseinrichtung (4) ein Grundelement (3) aufweist, wobei die mindestens zwei Messeinrichtungen (2) über jeweils mindestens eine Gelenkverbindung (21) mit mindestens zwei Rotationsfreiheitgraden mit dem Grundelement (3) gelenkig verbunden sind.

7. Vorrichtung nach Anspruch 6, **dadurch gekennzeichnet, dass** die mindestens zwei Messeinrichtungen (2) über jeweils mindestens eine Gelenkverbindung (21) mit drei Rotationsfreiheitgraden mit dem Grundelement (3) gelenkig verbunden sind.

8. Vorrichtung nach Anspruch 6 oder 7, **dadurch gekennzeichnet, dass** die jeweilige Gelenkverbindung (21) pro Rotationsfreiheitgrad ein separates Gelenk (50, 112) aufweist, wobei die Gelenke (50, 112) einer Gelenkverbindung (21) miteinander gekoppelt sind.

9. Vorrichtung nach Anspruch 6 oder 7, **dadurch gekennzeichnet, dass** die jeweilige Gelenkverbindung (21) mit einem bestimmten Rotationsfreiheitsgrad ein einzelnes Gelenk mit dem entsprechenden Rotationsfreiheitgrad aufweist.

10. Vorrichtung nach einem der Ansprüche 6 bis 9, **dadurch gekennzeichnet, dass** in der jeweiligen Gelenkverbindung (21) pro Freiheitsgrad eine Rückstellfeder vorgesehen ist, die die jeweiligen Messeinrichtung (2) wieder in eine Ausgangslage bezogen auf das Grundelement zurückstellen.

11. Vorrichtung nach einem der Ansprüche 1 bis 10, **dadurch gekennzeichnet, dass** der Abstand zwischen den Messeinrichtungen (2) einstellbar ist

12. Vorrichtung nach Anspruch 1, **dadurch gekennzeichnet, dass** in der Messkapsel (10) mindestens zwei Sensoren (57, 58) angeordnet sind, wobei die Sensoren (57, 58) beim Messen in unterschiedlichen Abständen von der zu untersuchenden Hautoberfläche angeordnet sind.

13. Gerät mit einer Vorrichtung nach einem der Ansprüche 1 bis 12, wobei das Gerät eine Auswerteeinheit aufweist, die mit der Vorrichtung (1) nach einem der Ansprüche 1 bis 12 verbunden ist und die Messergebnisse der Messeinrichtungen (2) erhält, wobei die Auswerteeinheit einen Durchschnittsmesswert aus den Messwerten der einzelnen Messeinrichtungen (2) berechnet und ausgibt.

14. Gerät mit einer Vorrichtung nach einem der Ansprüche 1 bis 12, wobei das Gerät eine Auswerteeinheit aufweist, die mit der Vorrichtung (1) nach einem der Ansprüche 1 bis 12 verbunden ist und die Messergebnisse der Messeinrichtungen (2) erhält, wobei die Auswerteeinheit die Messwerte der einzelnen Messeinrichtunen (2) einzeln ausgibt und anzeigt zu welcher Messeinrichtung das jeweilige Messergebnis gehört.

## Claims

1. Device (1) for measuring the amount of a substance given off from a skin surface (30) by diffusion, comprising
a measuring head adapted to be placed on the skin surface (30),
wherein the measuring head comprises at least two measuring means (2) spaced apart from each other, wherein the in total at least two measuring means (2) are movable independently of each other such that they can be adapted to the curvature of the skin surface (30) when being placed on the skin surface, wherein the respective measuring means (2) each comprise an open measuring capsule (10), wherein the measuring capsule (10) has at least two openings (11, 12), wherein one of the at least two openings (12) is adapted to be placed on the skin surface (30) to be examined, wherein at least one coupling means (4) is provided at a handle (14), and wherein the measuring means (2) are arranged on this coupling means (4).

2. Device of claim 1, **characterized in that** the respectively at least two measuring means (2) are movable independently of each other with respect to at least one rotational degree of freedom, so that, during measuring, all measuring means (2) can be placed on an uneven skin surface (30) at the same time.

3. Device of claim 1 or 2, **characterized in that** the respectively at least two measuring means (2) are movable independently of each other with respect to at least two rotational degrees of freedom.

4. Device of one of claims 1 to 3, **characterized in that** the respectively at least two measuring means (2) are movable independently of each other with respect to at least three rotational degrees of freedom.

5. Device of one of claims 1 to 4, **characterized in that** at least one returning element is provided which, after measuring, returns the measuring means (2) to the initial position, when they are not in contact with the sin surface (30) to be examined.

6. Device of claim 1, **characterized in that** the coupling means (4) comprises a base element (3), wherein the at least two measuring means (2) being hinged to the base element (3) via, respectively, at least one hinge joint (21) with at least two rotational degrees of freedom.

7. Device of claim 6, **characterized in that** the at least two measuring means (2) are hinged to the base element (3) via, respectively, at least one hinge joint (21) with three rotational degrees of freedom.

8. Device of claim 6 or 7, **characterized in that** the respective hinge joint (21) comprises one separate hinge (50, 112) per rotational degree of freedom, the hinges (50, 112) of a hinge joint (21) being coupled with each other.

9. Device of claim 6 or 7, **characterized in that** a respective hinge joint (21) having a defined rotational degree of freedom comprises a single hinge having a corresponding rotational degree of freedom.

10. Device of one of claims 6 to 9, **characterized in that** one return spring is provided in a respective hinge joint (21) per degree of freedom, which spring returns the respective measuring means (2) to an initial position with respect to the base element.

11. Device of one of claims 1 to 10, **characterized in that** the distance between the measuring means (2) is adjustable.

12. Device of claim 1, **characterized in that** at least two sensors (57, 58) are arranged in the measuring capsule (10), wherein, during measuring, the sensors (57, 58) are arranged at different distances from the skin surface to be examined.

13. Apparatus comprising a device of one of claims 1 to 12, wherein the apparatus comprises an evaluation unit which is connected with the device (1) of one of claims 1 to 12 and receives the measuring results of the measuring means (2), wherein the evaluating unit calculates and outputs an average value from the measuring values of the individual measuring means (2).

14. Apparatus comprising a device of one of claims 1 to 12, wherein the apparatus comprises an evaluation unit which is connected with the device (1) of one of claims 1 to 12 and receives the measuring results of the measuring means (2), wherein the evaluating unit individually outputs the measuring values of the individual measuring means (2) and displays the measuring unit associated to the respective measuring result.

## Revendications

1. Dispositif (1) destiné à mesurer la quantité d'une substance libérée par diffusion par une surface de la peau (30), avec une tête de mesure pouvant être placée sur la surface de la peau (30),
la tête de mesure comportant au moins deux équipements de mesure (2) qui sont disposés à distance l'un de l'autre, les au total au moins deux équipements de mesure (2) étant mobiles indépendamment l'un de l'autre de telle sorte qu'ils peuvent être adaptés à la courbure de la surface de la peau (30) lors du placement sur la surface de la peau, les équipements de mesure (2) respectifs comportant respectivement une capsule de mesure (10) ouverte, la capsule de mesure (10) comportant au moins deux ouvertures (11, 12), une des au moins deux ouvertures (12) pouvant être placée sur la surface de la peau (30) à examiner,
au moins un équipement d'accouplement (4) étant prévu sur une poignée (14), et
les équipements de mesure (2) étant disposés sur cet équipement d'accouplement (4).

2. Dispositif selon la revendication 1, **caractérisé en ce que** les respectivement au moins deux équipements de mesure (2) sont mobiles indépendamment l'un de l'autre par rapport à au moins un degré de liberté de rotation de telle sorte que, lors de la mesure, tous les équipements de mesure (2) peuvent être placés simultanément sur une surface de la peau (30) inégale.

3. Dispositif selon la revendication 1 ou 2, **caractérisé en ce que** les respectivement au moins deux équipements de mesure (2) sont mobiles indépendamment l'un de l'autre par rapport à au moins deux degrés de liberté de rotation.

4. Dispositif selon l'une des revendications 1 à 3, **caractérisé en ce que** les respectivement au moins deux équipements de mesure (2) sont mobiles indépendamment l'un de l'autre par rapport à au moins trois degrés de liberté de rotation.

5. Dispositif selon l'une des revendications 1 à 4, **caractérisé en ce qu'**il est prévu au moins un élément de rappel qui ramène dans la position initiale les équipements de mesure (2) après la mesure quand ils ne sont pas en contact avec la surface de la peau (30) à examiner.

6. Dispositif selon la revendication 1, **caractérisé en ce que** l'équipement d'accouplement (4) comporte un élément de base (3), les au moins deux équipements de mesure (2) étant raccordés de façon articulée à l'élément de base (3) par le biais respectivement d'au moins un raccordement articulé (21) avec au moins deux degrés de liberté de rotation.

7. Dispositif selon la revendication 6, **caractérisé en ce que** les au moins deux équipements de mesure (2) sont raccordés de façon articulée à l'élément de base (3) par le biais respectivement d'au moins un raccordement articulé (21) avec trois degrés de liberté de rotation.

8. Dispositif selon la revendication 6 ou 7, **caractérisé en ce que** le raccordement articulé (21) respectif comporte, par degré de liberté de rotation, une articulation (50, 112) séparée, les articulations (50, 112) d'un raccordement articulé (21) étant accouplées l'une à l'autre.

9. Dispositif selon la revendication 6 ou 7, **caractérisé en ce que** le raccordement articulé (21) respectif avec un degré de liberté de rotation défini comporte une articulation unique avec le degré de liberté de rotation correspondant.

10. Dispositif selon l'une des revendications 6 à 9, **caractérisé en ce que**, dans le raccordement articulé (21) respectif, il est prévu par degré de liberté de rotation un ressort de rappel qui ramène l'équipement de mesure (2) respectif dans une position initiale par rapport à l'élément de base.

11. Dispositif selon l'une des revendications 1 à 10, **caractérisé en ce que** la distance entre les équipements de mesure (2) est réglable.

12. Dispositif selon la revendication 1, **caractérisé en ce qu'**au moins deux capteurs (57, 58) sont disposés dans la capsule de mesure (10), les capteurs (57, 58) étant, lors de la mesure, disposés à des distances différentes de la surface de la peau à examiner.

13. Appareil avec un dispositif selon l'une des revendications 1 à 12, l'appareil comportant une unité d'analyse qui est raccordée au dispositif (1) selon l'une des revendications 1 à 12, et qui reçoit les résultats de mesure des équipements de mesure (2), l'unité d'analyse calculant et délivrant une valeur de mesure moyenne issue des valeurs de mesure des différents équipements de mesure (2).

14. Appareil avec un dispositif selon l'une des revendications 1 à 12, l'appareil comportant une unité d'analyse qui est raccordée au dispositif (1) selon l'une des revendications 1 à 12, et qui reçoit les résultats de mesure des équipements de mesure (2), l'unité d'analyse délivrant individuellement les valeurs de mesure des différents équipements de mesure (2) et indiquant à quel équipement de mesure (2) appartient le résultat de mesure respectif.
